# EUROPÄISCHE PATENTSCHRIFT

(11) **EP 1 614 433 B1**
(45) Veröffentlichungstag und Bekanntmachung des Hinweises auf die Patenterteilung: **14.12.2016**
(21) Anmeldenummer: 05011068.3
(22) Anmeldetag: 23.05.2005
(51) Int. Cl.: A61L 2/20, B65B 55/10

(54) **Verfahren zum Sterilisieren von Flaschen oder dgl. Behälter sowie Sterilisator zum Durchführen des Verfahrens**
Method of sterilising bottles or similar containers, and steriliser for carrying out the method
Procédé de stérilisation de bouteilles ou récipients similaires, et stérilisateur pour la mise en oeuvre de ce procédé

(30) Priorität: 26.06.2004 DE 102004030957
(43) Veröffentlichungstag der Anmeldung: 11.01.2006
(73) Patentinhaber: KHS GmbH, 44143 Dortmund (DE)
(72) Erfinder: Volker, Till, 65719 Hofheim/Taunus (DE)

(56) Entgegenhaltungen:
- WO-A-02/064174
- WO-A-03/022689
- WO-A-03/030950
- DE-A1- 3 339 930
- DE-A1- 19 949 692

## Beschreibung

Die Erfindung bezieht sich auf ein Verfahren gemäß Oberbegriff Patentanspruch 1. Weiterhin bezieht sich die Erfindung auf einen Sterilisator gemäß Oberbegriff Patentanspruch 7.

Beispielsweise in der Getränkeindustrie, d. h. beim Abfüllen von Getränken in Flaschen oder dgl. Behälter ist es vielfach erforderlich, diese Behälter vor dem Füllen zur Erzielung der erforderlichen Keimfreiheit und damit Haltbarkeit des abgefüllten Produktes an den Innenflächen zu sterilisieren.

Bekannt ist hierbei z.B. die sogenannte H2O2-Sterilisation. Bei diesem Verfahren wird flüssiges H2O2 zunächst feinzerstäubt einem Luftstrom beigemischt, wobei es sich in der Regel um einen Strom steriler Luft handelt. Anschließend wird dieses H2O2-Luft-Gemisch einem Verdampfer zugeführt, in welchem das noch flüssige H2O2 vollständig verdampft wird. Nachfolgend wird dieses Dampf-Luft-Gemisch in die zu sterilisierenden Behälter eingeleitet, wo das H2O2 sofort an den kalten Behälterinnenwandungen kondensiert und dort einen gleichmäßigen Flüssigkeitsfilm bildet. Zur nun folgenden Aktivierung des H2O2, d.h. zur Auslösung der Zersetzung des H2O2, ist es erforderlich, dieses auf eine bestimmte Temperatur zu erwärmen, bzw diesem eine bestimmte Wärmemenge zuzuführen, welche in der Regel durch ein Aktivierungsmedium auf das H2O2 übertragen wird. Bei diesem Aktivierungsmedium handelt es sich in den meisten Fällen um in die Behälter eingeblasene sterile Warmluft, welche bis auf die gewünschte Aktivierungstemperatur erwärmt wurde.

Im Laufe des Zersetzungsprozesses zerfällt das H2O2 in Wasser und freie Radikale, nämlich atomaren Sauerstoff und HO-Gruppen, welche die eigentliche Sterilisation im Wesentlichen durchführen. Nach dem Abschluss der Sterilisation werden die Behälter mittels Spül- und/oder Trockenluft ausgeblasen und getrocknet.

Da die Aktivierung des H2O2 mit steigender Temperatur des Aktivierungsmediums mit überproportional steigender Geschwindigkeit abläuft, wodurch sich deutliche Reduzierungen der Zykluszeiten erzielen lassen, ist es prinzipiell gewünscht, mit möglichst hohen Temperaturen zu arbeiten.

Speziell bei Flaschen oder Behältern aus Kunststoff, beispielsweise bei PET-Flaschen oder-Behältern, besteht nun das Problem, dass eine Überhitzung dieser Behälter über eine kritische Temperatur oder Grenztemperatur zu einer Zerstörung oder einer Verformung der Behälter führt und somit während des gesarnten Sterilisationsprozesses sicher vermieden werden muss. Bekannt sind daher auch Verfahren und Vorrichtung zur Sterilisation von PET-Flaschen (WO 03/022689 A1, WO 03/030950 A1, DE 199 49 692 A1), bei denen die als Aktivierungsmedium verwendete heiße Luft zwar mit einer Temperatur über der kritischen Temperatur oder Grenztemperatur, die bei PET-Flaschen im Bereich von etwa 55 bis 60°C liegt, in die jeweilige Flasche eingebracht wird, beispielsweise mit einer Temperatur über 75°C und unter 120°C, das Einbringen bzw. Einblasen der heißen Luft erfolgt dabei aber jeweils nur kurzzeitig oder impulsförmig, so dass ein Erhitzen der behandelten Flaschen über die Grenztemperatur nicht eintritt.

Bei bekannten Vorrichtungen bzw. Sterilisatoren für die H2O2-Sterilisation (WO 03/022689 A1, WO 03/030950 A1) ist vorgesehen, dass sämtliche an einem umlaufenden Rotor angeordnete Behandlungsstationen oder Sterilisatorköpfe von einer einzigen, gemeinsamen Einrichtung mit dem heißen Serilisationsmedium, d.h. mit dem heißen H2O2-Luft-Gemisch (H2O2-Nebel) versorgt werden, was schon wegen der relativ langen Verbindungen und deren Volumen u.a. Temperaturänderungen des in die Behälter eingebrachten Serilisationsmediums insbesondere auch bei Änderungen der Betriebsbedingungen (z.B. bei Änderung der Leistung) des Sterilisators zur Folge hat und keine befriedigende Lösung darstellt.

Bekannt sind weiterhin Verfahren sowie Vorrichtungen zum Sterilisieren von Behältern, insbesondere auch von Behältern, die aus Zuschnitten aus Flachmaterial, beispielsweise aus thermoplastisch beschichtetern Kartonzuschnitten hergestellt sind (DE-A-32 35 476, WO 00/00394). Für das Sterilisieren werden die Behälter mit Hilfe eines Linearförderers u.a. an einem Sterilisationskopf vorbeibewegt, an welchem in den jeweiligen Behälter ein erhitztes Sterilisationsmedium aus einer verdampften wässrigen Lösung von Wasserstoffperoxid (H2O2) eingebracht wird. Das Erhitzen des Sterilisationsmediums erfolgt in einem Heizkanal des Sterilisationskopfes. Die mit dem heißen Sterilisationsmedium beaufschlagten Behälter werden dann mit dem Transporteur an eine weitere Behandlungsstation oder einen weiteren Behandlungskopf bewegt, an welchen jeder Behälter mit einem heißen Aktivierungsmedium, beispielsweise mit heißer Luft beaufschlagt wird, die entweder von einer Heizeinrichtung an dem betreffenden Behandlungskopf oder aber von einer für mehrere Behandlungspositionen gemeinsamen Heizeinrichtung erzeugt wird. Nachteilig ist bei diesen bekannten Verfahren und Vorrichtungen, dass für das Sterilisieren jedes Behälters wenigstens zwei unterschiedliche Behandlungs- oder Sterilisationsköpfe oder -positionen erforderlich sind, nämlich ein Behandlunge- oder Sterilisationskopf zum Einbringen des heißen Sterilisationsmediums und ein weiterer Behandlungs- oder Sterilisationskopf zum Einbringen des heißen Aktivierungsmediums.

Aufgabe der Erfindung ist es, ein Verfahren aufzuzeigen, welches es ermöglicht, auch solche Behälter, die insbesondere wegen des verwendeten Behältermaterials temperaturempfindlich sind, d. h. über eine bestimmte Grenztemperatur nicht erhitzt werden dürfen, zuverlässig zu sterilisieren, und zwar mit ausreichend hoher Leistung (Anzahl der behandelten Behälter je Zeiteinheit). Zur Lösung dieser Aufgabe ist ein Verfahren entsprechend dem Patentanspruch 1 ausgebildet.

Bei dem erfindungsgemäßen Verfahren wird das Aktivierungsmedium auf eine Behandlungstemperatur erhitzt, welche deutlich über der Grenztemperatur der zu behandelnden Behälter liegt. Um eine umfassende und schnelle Aktivierung des H2O2 zu erreichen, aber dennoch eine Beschädigung der Behälter zu vermeiden, erfolgt das Einbringen des heißen Aktivierungsmediums in den jeweiligen Behälter während der Aktivierungsphase impulsförmig, beispielsweise einmalig oder aber mit Unterbrechungen mehrmals, wobei die Abgabedauer, während der das heiße Aktivierungsmedium in den jeweiligen Behälter einströmt, dann jeweils kürzer ist als die Gesamtdauer der Aktivierungsphase.

Das erfindungsgemäße Verfahren eignet sich insbesondere aber nicht ausschließlich für die H2O2-Sterilisation von PET- oder Kunststoffflaschen oder dgl. Behälter, deren kritische Grenztemperatur etwa zwischen 55 und 60°C liegt, wobei die Behälter zur Vermeidung eines Schrumpfens oder Verformens nicht über diese Grenztemperatur hinaus erhitzt werden dürfen.

Die Behandlungs- oder Abgabetemperatur des heißen Aktivierungsmediums liegt bei dem erfindungsgemäßen Verfahren beispielsweise im Bereich zwischen 130 und 150°C.

Der Erfindung liegt die Erkenntnis zugrunde, dass die Aktivierungs- bzw. Zerfallsgeschwindigkeit des H2O2 mit steigender Temperatur, bzw. mit steigender Temperatur des Aktivierungsmediums exponentiell wächst, wohingegen der Wärmeeintrag in die Behälter und somit auch deren Temperatur mit der Temperatur des Aktivierungsmediums lediglich linear wächst.

Durch die, im Rahmen der vorliegenden Erfindung ebenfalls vorgesehene, gepulste Abgabe des hocherhitzten Aktivierungsmediums, wird bei weiterhin optimierter Aktivierungsgeschwindigkeit der Wärmeeintrag in die Behälter weiter vermindert, so dass diese nicht über ihre Grenztemperatur hinaus erhitzt werden.

Ein Sterilisator zum Durchführen des Verfahrens ist Gegenstand des Patentanspruches 7.

Weiterbildungen der Erfindung sind Gegenstand der Unteransprüche. Die Erfindung wird im Folgenden anhand der Figuren an einem Ausführungsbeispiel näher erläutert. Es zeigen:
- Fig. 1: in vereinfachter Darstellung eine Draufsicht auf einen um eine vertikale Maschinenachse umlaufenden Rotor eines Sterilisators für Flaschen (Kunststoff-Flaschen, beispielsweise PET-Flaschen) oder dgl. Behälter mit einem am Umfang dieses Rotors vorgesehenen Sterilisator- oder Verdampferkopf;
- Fig. 2 und 3: Schnitte entsprechend den Linien I-I bzw. II-II der Figur 1.

Der in den Figuren allgemein mit 1 bezeichnete Sterilisator, von dem lediglich ein um eine vertikale Maschinenachse umlaufend angetriebener Rotor 2 sowie ein Sterilisatorkopf 3 dargestellt ist, dient zum Sterilisieren (H2O2-Sterilisation) von temperaturempfindlichen Behältern, nämlich PET-Flaschen 4 mit einem heißen Sterilisationsmedium, welches aus einem Luft-Wasser-Aerosol durch Erhitzen gebildet ist.

Unter den Sterilisatorköpfen 3, die in gleichmäßigen Winkelabständen am Umfang des Rotors 2 angeordnet sind, ist jeweils ein Behälter- oder Flaschenträger 2.1 vorgesehen, an dem die jeweilige, zu sterilisierende Flasche z.B. hängend gehalten ist, und zwar achsgleich mit einer vertikalen Sterilisatorkopfachse KA, sodass beim Aktivieren des Sterilisatorkopfes 3 ein Strahl des heißen Sterilisationsmedium in den Innenraum der sich unterhalb des Sterilisationskopfes 3 befindlichen zu sterilisierenden Flasche 4 Flasche ausgebracht werden kann.

Der Sterilisatorkopf 3 besteht aus einem Erhitzer oder Wärmetauscher 5 mit einem Gehäuse 6, in welchem u. a. ein die Achse KA schraubenartig umschließender Strömungs- oder Heizkanal 7 ausgebildet ist, und zwar bei der dargestellten Ausführungsform dadurch, dass in eine kreiszylinderförmige, achsgleich mit der Achse KA ausgebildete durchgehende Öffnung des Gehäuses 6 ein Kern 8 eingesetzt ist, der an seinem Umfang eine den Strömungskanal 7 bildende schraubenförmige Nut aufweist.

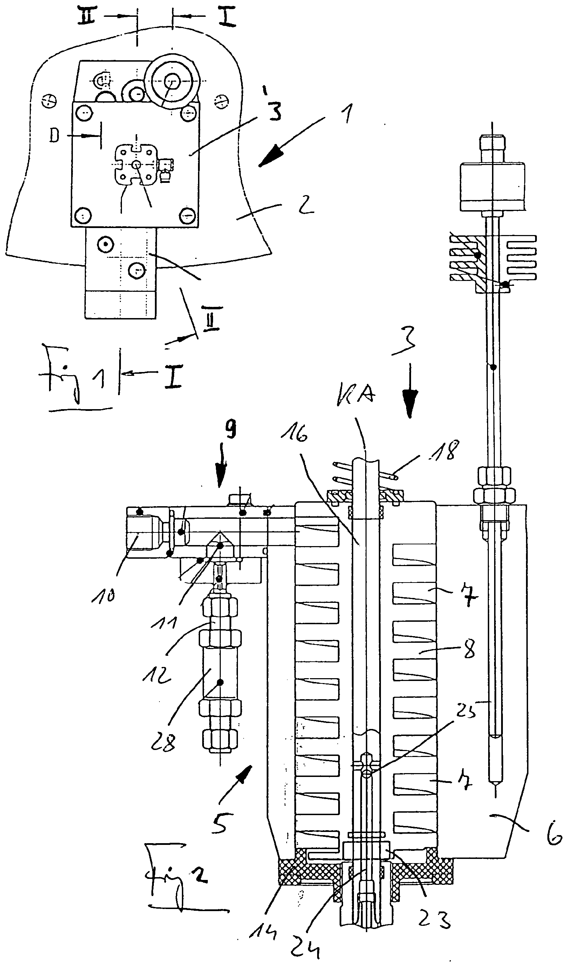

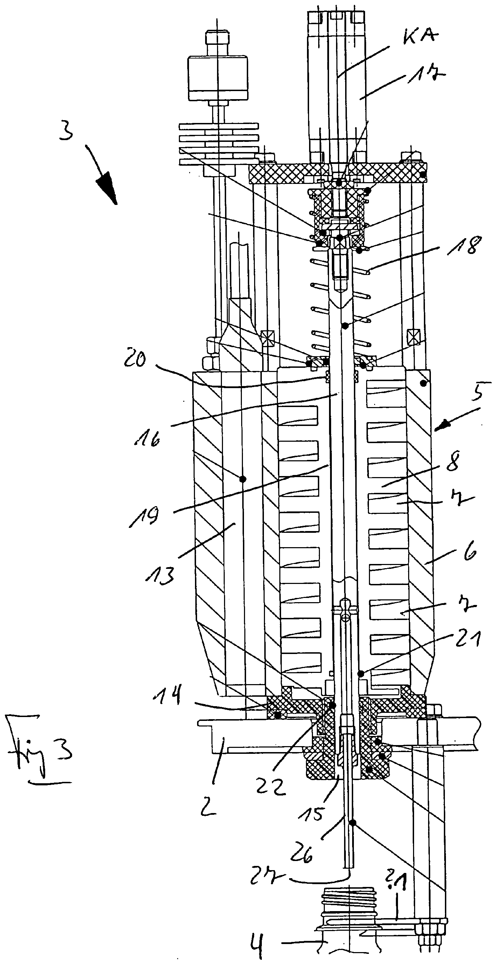

Das in den Figuren 2 und 3 obere Ende des nach außen hin geschlossenen Strömungskanal 7 steht mit einer Sprüheinrichtung 9 in Verbindung, der über den Anschluss 10 sterile Druckluft zugeführt wird und die eine Sprühdüse 11 besitzt, der über den Anschluss 12 und eine mit diesem Anschluss verbundene, nicht dargestellte Pumpe, beispielsweise einer Membran-Pumpe, H2O2 zugeführt wird, welches dann zur Bildung des H2O2-Luft-Aerosols über die Sprühdüse 11 als fein versprühter Strahl oder Nebel in den die Sprüheinrichtung 9 durchströmenden Luftstrom eingebracht wird, sodass dieses aus Luft und H2O2 bestehende Aerosol dann in den Strömungskanal 7 des Wärmetauschers 5 gelangt.
Durch eine Heizeinrichtung 13, die bei der dargestellten Ausführungsform von einer elektrischen Heizpatrone mit Thermofühler gebildet ist, werden der Wärmetauscher 5 und insbesondere auch der Kern 8 beheizt, und zwar auf eine Temperatur, die deutlich über der Grenztemperatur liegt, auf welche die Flaschen 4 ohne ein Beschädigen oder Verformen erhitzt werden dürfen. Mit dem Heizelement 13 erfolgt beispielsweise eine Erhitzung des Kernes 8 auf eine Temperatur im Bereich zwischen etwa 130-150°C.

Das untere Ende des Gehäuses 6 ist durch ein Abschlusselement 14 abgeschlossen, mit welchem der jeweilige Sterilisatorkopf 3 bei der dargestellten Ausführungsform auch an dem Rotor 2 befestigt ist und in welchem ein achsgleich mit der Achse KA angeordneter und an der Unterseite des Abschlusselementes 14 offener Führungskanal 15 für eine Stange 16 ausgebildet ist. Diese definiert mit ihrer Achse die Achse KA und ist durch eine Betätigungseinrichtung 17, die oberhalb des Wärmetauschers 5 vorgesehen ist, aus der in den Figuren 2 und 3 dargestellten angehobenen Ausgangsstellung gegen die Wirkung einer Rückstellfeder 18 in Richtung der Achse AK in eine Arbeitsposition nach unten bewegbar.

Die Stange 16 ist weiterhin auch in einer achsgleich mit der Achse KA ausgebildeten Bohrung 19 im Kern 8 geführt, und zwar abgedichtet durch Dichtungen 20 im Bereich der Oberseite des Kernes 8 und durch eine von einem O-Ring gebildete Dichtung 21 im unteren Bereich des Kernes 8. Durch eine weitere, bei der dargestellten Ausführungsform ebenfalls von einem O-Ring gebildete Dichtung 22 ist die Stange abgedichtet in der Öffnung 16 geführt. Im unteren Bereich bildet der Kern 8 eine die Stange 16 umschließende Kammer 23, die mit dem unteren Ende des Strömungskanals 7 in Verbindung steht.
In dem unteren Teilbereich der Stange 16 ist ein achsgleich mit der Achse dieser Stange angeordneter und an der Unterseite der Stange offener Kanal 24 gebildet, der an seinem oberen Ende über von Querbohrungen gebildeten Steueröffnungen 25 am Umfang der Stange 16 offen ist. Bei dem in den Figuren 2 und 3 dargestellten Zustand befinden sich die Steueröffnungen 25 innerhalb der Bohrung 19, sodass eine Strömungsverbindung aus der Kammer 23 über die Steueröffnungen 25 in den Kanal 24 nicht besteht. Wird die Stange 16 durch die Betätigungseinrichtung 17 nach unten in ihre aktivierte Stellung oder Arbeitsposition bewegt, so befinden sich die Steueröffnungen 25 in der Kammer 23, wodurch dann eine Strömungsverbindung aus dieser Kammer über die Steueröffnungen 25 in den Kanal 24 besteht.
An dem unteren Ende der Stange 16 ist auswechselbar ein Rohr 26 befestigt, sodass dieses achsgleich mit der Achse der Stange 16 liegt und über die Unterseite des Sterilisatorkopfes 3 bzw. des Abschlusses 14 vorsteht. Nach dem Befestigen des Rohres 26 ist der in dem Rohr ausgebildete Kanal mit dem Kanal 24 verbunden.

Für das Sterilisieren der Flaschen 4 werden zunächst die Wärmetauscher 5 der Wärmetauscherköpfe 3 über ihre elektrischen Heizeinrichtungen 13 auf die erforderliche Behandlungstemperatur, beispielsweise auf die Temperatur zwischen 130 und 150°C erhitzt.
Die zu sterilisierenden Flaschen 4 werden dann an einer Flaschenaufnahme jeweils einzeln an einen Flaschenhalter 2.1 des umlaufenden Rotors 2 übergeben und nach dem Sterilisieren an einem Flaschenauslauf vom Rotor abgenommen bzw. an einer nachgeschaltete Füllmaschine weiter geleitet. Während der Drehbewegung des Rotors zwischen dem Flascheneinlauf und Flaschenauslauf wird gesteuert die jeweilige Betätigungseinrichtung 17 aktiviert, sodass die Stange 26 und mit dieser auch das jeweilige Rohr 26 in die Flasche 4 eingeführt werden, sodass über den dann mit der Kammer 23 über die Öffnungen 25 in Verbindung stehenden Kanal 24 und den im Rohr 26 ausgebildeten Rohrkanal das in der Sprüheinrichtung 9 erzeugte und beim Durchströmen des schrauben- oder wendelartigen Strömungskanals 7 erhitze Sterilisationsmedium, welches aus heißer Luft und insbesondere aus verdampftem H2O2 besteht, über die am unteren Ende des Rohres 26 gebildete Abgabeöffnung 27 in die jeweilige Flasche 4 eingebracht wird.

Da für den jeweiligen Wärmetauscher 5 eine relativ hohe Betriebstemperatur gewählt ist (im Bereich von etwa 130 - 150° C) ist es möglich, den Anteil an H2O2, welches über die Sprüheinrichtung 9 in den Luftstrom eingebracht wird, relativ hoch zu halten und damit auch die je Zeiteinheit im Strömungskanal 7 erzeugte Menge an heißem Sterilisationsmedium, d.h. Luft und H2O2-Dampf, welches in die jeweilige Flasche 4 eingebracht wird.

Um eine Beschädigung der Flaschen 4 zu vermeiden und dennoch eine für eine optimale Aktivierung des H2O2 notwendige Wärmemenge hoher Temperatur in die Flaschen 4 einzubringen, erfolgt während des Aktivierungsprozesses eine pulsierende Abgabe des heißen Aktivierungsmediums, wofür durch entsprechendes impulsförmiges Aktivieren der Betätigungseinrichtung 17 beispielsweise die jeweilige Stange 16 mehrfach zwischen ihrer Ausgangsstellung und ihrer aktivierten Stellung bewegt wird. Da weiterhin die Luft über den Anschluss 10 mit Druck zugeführt wird und sich hierdurch insbesondere bei nicht aktiviertem Sterilisatorkopf, d. h. immer dann, wenn sich die Stange 16 in ihrer Ausgangsstellung befindet, im Strömungskanal 7 ein erhöhter Druck aufbaut, ist eine erhöhte Enthalpie bzw. Wärmemenge insbesondere jedoch eine erhöhte Temperatur für das heiße Aktivierungsmedium erzielbar.

Nach den Sterilisieren erfolgt jeweils ein Ausblasen der Flasche 4 zum Entfernen von Kondensat oder Wasserresten. Mit 28 ist noch ein Steuerventil in der Versorgungsleitung der Sprühdüse 11 bezeichnet, mit dem die H2O2-Versorgung an diese Düse unterbrochen werden kann.

Die Erfindung wurde voranstehend an einem Ausführungsbeispiel beschrieben. Es versteht sich, dass zahlreiche Änderungen sowie Abwandlungen möglich sind, ohne dass dadurch der der Erfindung zugrunde liegende Erfindungsgedanke verlassen wird. So wurde vorstehend beschrieben, dass das Beheizen des Wärmetauschers 5 mittels der elektrischen Heizeinrichtung 13 (Heizpatrone mit Temperaturfühler) erfolgt. Es bestehen aber auch andere Möglichkeiten, beispielsweise ein Beheizen mit Hilfe von Dampf.

### Bezugszeichenliste

- 1: Sterilisator
- 2: Rotor
- 2.1: Behälterträger
- 3: Sterilisatorkopf
- 4: PET-Flaschen
- 5: Wärmetauscher
- 6: Gehäuse
- 7: Strömungskanal
- 8: Kern
- 9: Einrichtung
- 10: Anschluss
- 11: Düse
- 12: Anschluss
- 13: Heizeinrichtung
- 14: Abschlusselement
- 15: Führungskanal
- 16: Stange
- 17: Betätigungseinrichtung
- 18: Rückstellfeder
- 19: Bohrung
- 20, 21, 22: Dichtung
- 23: Kammer
- 24: Bohrung
- 25: Steueröffnung
- 26: Rohr
- 27: Abgabeöffnung
- 28: Steuerventil

- KA: Sterilisatorkopfachse

## Patentansprüche

1. Verfahren zum H2O2-Sterilisieren von Flaschen oder dergleichen Behältern (4) unter Verwendung eines Sterilisators mit mehreren in gleichmäßigen Winkelabständen an einem um eine vertikale Maschinenachse umlaufenden Rotor (2) vorgesehenen Sterilisatorköpfen (3) zum Einbringen von erhitztem H2O2-Luft-Aerosol als Sterilisationsmedium über einen in einem Rohr (26) ausgebildeten Rohrkanal in den jeweiligen Behälter (4) und zum anschließenden Aktivieren des eingebrachten H2O2-Luft-Aerosols durch Ausblasen der Behälter (4) mit einem Aktivierungsmedium, welches in einem Heizkanal (7) eines Wärmetauschers (5) auf eine Temperatur über einer Grenztemperatur erhitzt wird, bei der ein Verformen oder eine Zerstörung der Behälter eintreten würde, wobei das heiße Aktivierungsmedium aber zum Aktivieren impulsartig in den jeweiligen Behälter(4) so eingebracht wird, dass die Behältertemperatur unterhalb der Grenztemperatur bleibt, wobei die Sterilisatorköpfe (3) am Umfang des Rotors (2) in gleichmäßigen Winkelabständen angeordnet sind, **dadurch gekennzeichnet, dass** jeder Sterilisatorkopf (3) einen Wärmetauscher (5) umfasst, wobei während der Drehbewegung des Rotors (2) zwischen einem Flascheneinlauf und Flaschenauslaufdurch Aktivieren einer Betätigungseinrichtung (17) das jeweilige an einer Stange (16) der Betätigungseinrichtung (17) vorgesehene Rohr (26) in den Behälter (4) eingeführt und erst hierbei der Rohrkanal über Steueröffnungen (25) eines in der Stange (16) ausgebildeten Kanals (24) mit einer mit dem Heizkanal (7) in Verbindung stehenden Kammer (23) verbunden wird, so dass über das Rohr (26)
- einerseits das erhitzte H2O2-Luft-Aerosol, welches durch Einsprühen von H2O2 in einen Luftstrom in einer mit dem jeweiligen Sterilisatorkopf (3) verbundenen und mit diesem umlaufenden Sprüheinrichtung (9) und durch Einleiten des so gebildeten H2O2-Luft-Aerosol in den Heizkanal (7) erzeugt wird, und
- andererseits in dem Aktivierungsprozessals Aktivierungsmedium heiße Luft aus den Heizkanal (7) impulsartig durch Betätigung der Betätigungseinrichtung in den jeweiligen Behälter (4) geleitet werden.

2. Verfahren nach Anspruch 1, **dadurch gekennzeichnet, dass** das Aktivierungsmedium in einem in jedem Sterilisatorkopf (3) eigenständig vorgesehenen Heizkanal (7) des dortigen Wärmetauschers (5) erhitzt wird, der ein eine Achse des Sterilisatorkopfes schraubenförmig umschließender Kanal ist.

3. Verfahren nach Anspruch 1 oder 2, **dadurch gekennzeichnet, dass** das Aktivierungsmedium in dem jeweiligen Heizkanal (7) auf eine Temperatur im Bereich zwischen etwa 130 - 150°C erhitzt wird.

4. Verfahren nach Anspruch 1 bis 3, **dadurch gekennzeichnet, dass** die Behälter PET-Flaschen (4), insbesondere PET-Flaschen mit einer Grenztemperatur zwischen etwa 55 und 60°C sind.

5. Verfahren nach einem der vorhergehenden Ansprüche, **dadurch gekennzeichnet, dass** während der Behandlungsdauer das Einleiten des heißen Aktivierungsmediums wenigstens einmal unterbrochen wird.

6. Verfahren nach einem der vorhergehenden Ansprüche, **dadurch gekennzeichnet, dass** das Einbringen des heißen Aktivierungsmediums in die jeweiligen Behälter (4) durch ein in den Behälter (4) eingeführtes Rohr des jeweiligen Sterilisatorkopfes (3) erfolgt.

7. Sterilisator zum H2O2-Sterilisieren von Flaschen oder dgl. Behältern (4),
mit mehreren an einem um eine vertikale Maschinenachse umlaufenden Rotor (2) vorgesehenen Sterilisatorköpfen (3), welche in gleichmäßigen Winkelabständen am Umfang des Rotors (2) angeordnet sind, zum Einbringen von H2O2-Luft-Aerosol als Sterilisationsmedium in den jeweiligen an einem Sterilisatorkopf (3) angeordneten Behälter (4) und zum anschließenden Aktivieren des eingebrachten H2O2 durch Ausblasen der Behälter (4) mit einem in einem Heizkanal (7) eines Wärmetauschers (5)erhitztes Aktivierungsmedium, sowie mit einer Ventilsteuerung (16, 17, 23, 24, 25) zur zeitlich gesteuerten Abgabe des heißen Aktivierungsmediums über eine Abgabeöffnung (27) in das Innere des zu sterilisierenden Behälters (4), wobei die Ventilsteuerung eine Steuerventilanordnung (16, 23, 24, 25) und eine Betätigungseinrichtung (17) aufweist, die für ein impulsförmiges Öffnen und Schließen der Ventilanordnung (16, 23, 24, 25) ausgebildet ist,
**dadurch gekennzeichnet, dass**
jeder Sterilisatorkopf (3) einen Wärmetauscher (5) umfasst, dessen Heizkanal mit einer mit dem jeweiligen Sterilisatorkopf (3) umlaufenden Sprüheinrichtung (9) verbundenen ist, der über einen Anschluss (10) sterile Druckluft zugeführt wird und die eine Sprühdüse (11) besitzt, der über einen weiteren Anschluss (12) H2O2 von einer Pumpe zugeführt wird, und dass der Wärmetauscher (5) jedes Sterilisatorkopfes (3) zum Erhitzen des Aktivierungsmediums auf eine Temperatur über einer Grenztemperatur ausgebildet ist, bei der eine Zerstörung oder Verformung der Behälter eintreten würde.

8. Sterilisator nach Anspruch 7, **dadurch gekennzeichnet, dass** der jeweilige Heizkanal (7) ein eine Achse des Sterilisatorkopfes (3) schraubenförmig umschließender Kanal ist.

9. Sterilisator nach Anspruch 7 oder 8, **gekennzeichnet durch** eine von der Betätigungseinrichtung (17) in einer Achse parallel zu einer vertikalen Sterilisatorkopfachse (KA) zwischen einer Ausgangsposition und einer Arbeitsposition axial bewegbaren Stange (16), in der ein mit der Abgabeöffnung (27) in Verbindung stehender axialer Kanal (24) ausgebildet ist, der an einem der Abgabeöffnung (27) entfernt liegenden Ende wenigstens eine Steueröffnung (25) aufweist, die den axialer Kanal (24) erst bei in der Arbeitsposition befindlicher Stange (16) mit dem Heizkanal (7) oder mit einer mit diesem Kanal in Verbindung stehenden Kammer (23) verbindet.

10. Sterilisator nach einem der Ansprüche 7 - 9, **dadurch gekennzeichnet, dass** der Wärmetauscher (5) jedes Sterilisatorkopfes (3) zum Erhitzen des Aktivierungsmediums auf eine Temperatur über 55 und 60°C, bevorzugt auf eine Temperatur im Bereich zwischen etwa 130 - 150°C ausgebildet ist.

11. Sterilisator nach einem der Ansprüche 7 - 10, **dadurch gekennzeichnet, dass** jeder Sterilisatorkopf (3) eigenständig eine Sprüheinrichtung (9) mit Sprühdüse (11) zum Einsprühen von H2O2 in einen anschließend den Heizkanal (7) durchströmenden Luftstrom aufweist.

12. Sterilisator nach einem der Ansprüche 7 - 11, **dadurch gekennzeichnet, dass** im Gehäuse (6) ein Kern (8) aufgenommen ist, der an seinem Umfang wenigstens eine den Heizkanal (7) bildende Nut aufweist.

13. Sterilisator einem der Ansprüche 7 - 12, **dadurch gekennzeichnet, dass** das die Abgabeöffnung (27) bildende Rohr (26) mit einem der Abgabeöffnungen entfernt liegenden Ende an einem Ende der Stange (16) vorzugsweise austauschbar befestigt ist.

14. Sterilisator nach einem der Ansprüche 7 - 13, **gekennzeichnet durch** eine Heizeinrichtung (13), beispielsweise eine elektrische Heizeinrichtung oder Heizpatrone zum Beheizen des Heizkanals (7).

## Claims

1. Method for the H₂O₂ sterilisation of bottles or like containers (4) making use of a steriliser with a plurality of steriliser heads (3) provided on a rotor (2) circulating around a vertical machine axis, for introducing heated H₂O₂-air aerosol as the sterilisation medium, via a tube channel formed in a tube (26), into the respective container (4) and to the following activation of the introduced H₂O₂-air aerosol by blowing out the containers (4) with an activation medium, which is heated in a heating channel (7) of a heat exchanger (5) to a temperature above a limiting temperature at which deformation or destruction of the containers would occur, wherein, however, the hot activation medium is introduced in a pulsed manner into the respective container (4) for the activation, in such a way that the container temperature remains below the limiting temperature, wherein the steriliser heads (3) are arranged at uniform angular distances about the circumference of the rotor (2),
**characterised in that**
each steriliser head (3) comprises a heat exchanger (5), wherein, during the rotational movement of the rotor (2) between a bottle inlet and a bottle outlet, by activation of a actuation device (17), the tube (26) provided in each case at a rod (16) of the actuation device (17) is introduced into the container (4), and only thereby is the tube channel connected, via control openings (25) of a channel (24) formed in the rod (16), to a chamber (23) which is in communication with the heating channel (7), such that, via the tube (26):
- on the one hand, the heated H₂O₂ aerosol is produced, by the spraying of H₂O₂ into an air flow in a spray device (9), connected to and circulating with the respective steriliser head (3), and then by introducing the H₂O₂-air aerosol formed in this way into the heating channel (7), and
- on the other, in the activation process, the activation medium of hot air is delivered out of the heating channel (7) into the respective container (4) in a pulsed manner by the activation of the actuation device.

2. Method according to claim 1, **characterised in that** the activation medium is heated in a heating channel (7), provided independently in each steriliser head (3), of the heat exchanger (5) located there, this channel being a channel which surrounds an axis of the steriliser head in a helical manner.

3. Method according to claim 1 or 2, **characterised in that** the activation medium is heated in the respective heating channel (7) to a temperature in the range between some 130 - 150 °C.

4. Method according to claims 1 to 3, **characterised in that** the containers are PET bottles (4), in particular PET bottles with a limiting temperature of between some 55 and 60 °C.

5. Method according to any one of the preceding claims, **characterised in that**, during the treatment period, the introduction of the hot activation medium is interrupted at least once.

6. Method according to any one of the preceding claims, **characterised in that** the introduction of the hot activation medium into the respective containers (4) takes place through a tube of the respective steriliser head (3) introduced into the container (4).

7. Steriliser for the H₂O₂ sterilising of bottles or like containers (4), with a plurality of steriliser heads (3) provided on a rotor (2) circulating about a vertical machine axis, which are arranged at uniform angular distances about the circumference of the rotor (2), for the introduction of H₂O₂-air aerosol as sterilisation medium into the respective container (4) arranged at a steriliser head (3), and then the activation of the introduced H₂O₂ by blowing out the containers (4) with an activation medium heated in a heating channel (7) of a heat exchanger (5), and with a valve control (16, 17, 23, 24, 25) for the time-controlled delivery of the hot activation medium via a delivery opening (27) into the interior of the container (4) which is to be sterilised, wherein the valve control comprises a control valve arrangement (16, 23, 24, 25) and an actuation device (17), which is configured for a pulsed opening and closing of the valve arrangement (6, 23, 24, 25),
**characterised in that**
each steriliser head (3) comprises a heat exchanger (5), of which the heating channel is connected to a spray device (9), circulating about the respective steriliser head (3), into which device sterile compressed air is delivered via a connection (10) and which has a spray nozzle (11), into which H₂O₂ is delivered by a pump via a further connection (12), and that the heat exchanger (5) of each steriliser head (3) is configured for the heating of the activation medium to a temperature above the limiting temperature at which the destruction or deformation of the containers would occur.

8. Steriliser according to claim 7, **characterised in that** the respective heating channel (7) is a channel surrounding an axis of the steriliser head (3) in a helical manner.

9. Steriliser according to claim 7 or 8, **characterised by** a rod (16), which is axially movable by the actuation device (17) in an axis parallel to a vertical steriliser head axis (KA) between an initial position and a working position, in which an axial channel (24) is formed, which is in connection with the delivery opening (27) and which comprises at least one control opening (25) at an end located remotely from the delivery opening (27), this opening connecting the axial channel (24) to the heating channel (7), or to a chamber (23) in connection with this channel, only when the rod (16) is located in the working position.

10. Steriliser according to any one of claims 7 - 9, **characterised in that** the heat exchanger (5) of each steriliser head (3) is configured for heating the activation medium to a temperature above 55 and 60 °C, preferably to a temperature in the range between some 130 - 150 °C.

11. Steriliser according to any one of claims 7 - 10, **characterised in that** each steriliser head (3) independently comprises a spray device (9) with a spray nozzle (11) for spraying H₂O₂ into an air flow which then flows through the heating channel (7).

12. Steriliser according to any one of claims 7 - 11, **characterised in that** a core (8) is accommodated in the housing (6), which exhibits at its circumference at least one groove forming the heating channel (7).

13. Steriliser according to any one of claims 7 - 12, **characterised in that** the tube (26) forming the delivery opening is secured to an end of the rod (16), at an end located remotely from the delivery openings (27), preferably in an exchangeable manner.

14. Steriliser according to one of claims 7 - 13, **characterised by** a heating device (13), such as an electrical heating device or heating cartridge, for heating the heating channel (7).

## Revendications

1. Procédé servant à stériliser au H2O2 des bouteilles ou des contenants (4) similaires en utilisant un stérilisateur avec plusieurs têtes de stérilisateur (3) prévues, à des distances angulaires régulières, au niveau d'un rotor (2) tournant autour d'un axe de machine vertical, servant à mettre en place dans le contenant (4) respectif un aérosol de H2O2 et d'air chauffé en tant que milieu de stérilisation par l'intermédiaire d'un canal tubulaire réalisé dans un tube (26) et servant à activer ensuite l'aérosol d'H2O2 et d'air mis en place en purgeant par soufflage les contenants (47) avec un milieu d'activation, qui est chauffé à une température supérieure à une température limite dans un canal de chauffage (7) d'un échangeur de chaleur (5), température limite à laquelle une déformation ou une destruction des contenants surviendrait, dans lequel le milieu d'activation très chaud est, toutefois aux fins de l'activation, mis en place par impulsions dans le contenant (4) respectif de telle sorte que la température de contenant reste inférieure à la température limite, dans lequel les têtes de stérilisateur (3) sont disposées à des distances angulaires régulières au niveau de la périphérie du rotor (2), **caractérisé en ce que** chaque tête de stérilisateur (3) comprend un échangeur de chaleur (5), dans lequel, au cours du déplacement en rotation du rotor (2) entre une arrivée de bouteilles et une sortie de bouteilles, le tube (26) respectif prévu au niveau d'une tige (16) d'un système d'activation (17) est introduit, par l'activation d'un système d'activation (17), dans le contenant (4) et que seulement ici le canal tubulaire est relié, par l'intermédiaire d'ouvertures de commande (25) d'un canal (24) réalisé dans la tige (16), à une chambre (23) se trouvant en liaison avec le canal de chauffage (7) de sorte que, par l'intermédiaire du tube (26),
- d'une part l'aérosol de H2O2 et d'air chauffé soit produit par l'injection de H2O2 dans un flux d'air dans un système d'injection (9) relié à la tête de stérilisateur (3) respective et tournant avec cette dernière et par l'acheminement de l'aérosol de H2O2 et d'air ainsi formé à l'intérieur du canal de chauffage (7), et
- d'autre part, en tant que milieu d'activation, de l'air très chaud soit acheminé, dans le processus d'activation, hors du canal de chauffage (7), par impulsions, par l'actionnement du système d'actionnement, dans le contenant (4) respectif.

2. Procédé selon la revendication 1, **caractérisé en ce**
**que** le milieu d'activation est chauffé dans un canal de chauffage (7), prévu de manière autonome dans chaque tête de stérilisateur (3), de l'échangeur de chaleur (5) situé à cet endroit, lequel est un canal renfermant sous une forme de vis un axe de la tête de stérilisateur.

3. Procédé selon la revendication 1 ou 2, **caractérisé en ce que** le milieu d'activation est chauffé, dans le canal de chauffage (7) respectif, à une température située dans la plage comprise entre environ 130 - 150 °C.

4. Procédé selon la revendication 1 à 3, **caractérisé en ce que** les contenants sont des bouteilles en PET (4), en particulier des bouteilles en PET présentant une température limite comprise entre environ 55 et 60 °C.

5. Procédé selon l'une quelconque des revendications précédentes, **caractérisé en ce qu'**au cours de la durée de traitement, l'acheminement du milieu d'activation très chaud est interrompu au moins une fois.

6. Procédé selon l'une quelconque des revendications précédentes, **caractérisé en ce que** la mise en place du milieu d'activation très chaud dans les contenants (4) respectifs est effectuée par un tube, introduit dans le contenant (4), de la tête de stérilisateur (3) respective.

7. Stérilisateur servant à stériliser au H2O2 des bouteilles ou des contenants (4) similaires, comprenant plusieurs têtes de stérilisateur (3) prévues au niveau d'un rotor (2) tournant autour d'un axe de machine vertical, lesquelles sont disposées à des distances angulaires régulières au niveau de la périphérie du rotor (2), servant à mettre en place un aérosol de H2O2 et d'air en tant que milieu de stérilisation dans le contenant (4) respectif disposé au niveau d'une tête de stérilisateur (3) et servant à activer ensuite le H2O2 mis en place en purgeant par soufflage les contenants (4) avec un milieu d'activation chauffé dans un canal de chauffage (7) d'un échangeur de chaleur (5), ainsi que comprenant une commande de soupape (16, 17, 23, 24, 25) servant à distribuer de manière commandée dans le temps le milieu d'activation très chaud par l'intermédiaire d'une ouverture de distribution (27) dans l'intérieur du contenant (4) à stériliser, dans lequel la commande de soupape présente un ensemble de soupapes de commande (16, 23, 24, 25) et un système d'actionnement (17), qui est réalisé pour une ouverture et une fermeture par impulsions de l'ensemble de soupapes (16, 23, 24, 25),
**caractérisé en ce**
**que** chaque tête de stérilisateur (3) comprend un échangeur de chaleur (5), dont le canal de chauffage est relié à un système de pulvérisation (9) tournant avec la tête de stérilisateur (3) respective, auquel de l'air comprimé stérile est amené par l'intermédiaire d'un raccord (10) et qui possède une buse de pulvérisation (11), à laquelle du H2O2 provenant d'une pompe est amené par l'intermédiaire d'un autre raccord (12), et en ce que l'échangeur de chaleur (5) de chaque tête de stérilisateur (3) est réalisé afin de chauffer le milieu d'activation à une température supérieure à une température limite, à laquelle une destruction ou une déformation des contenants surviendrait.

8. Stérilisateur selon la revendication 7, **caractérisé en ce que** le canal de chauffage (7) respectif est un canal renfermant sous la forme d'une vis un axe de la tête de stérilisateur (3).

9. Stérilisateur selon la revendication 7 ou 8, **caractérisé par** une tige (16) pouvant être déplacée de manière axiale par le système d'actionnement (17) dans un axe de manière parallèle par rapport à un axe de tête de stérilisateur (KA) vertical entre une position de départ et une position de fonctionnement, dans laquelle un canal (24) axial se trouvant en liaison avec l'ouverture de distribution (27) est réalisé, lequel présente, au niveau d'une extrémité située de manière éloignée de l'ouverture de distribution (27), au moins une ouverture de commande (25), qui relie le canal (24) axial seulement, lorsque la tige (16) se trouve dans la position de fonctionnement, au canal de chauffage (7) ou à une chambre (23) se trouvant en liaison avec ledit canal.

10. Stérilisateur selon l'une quelconque des revendications 7 - 9, **caractérisé en ce que** l'échangeur de chaleur (5) de chaque tête de stérilisateur (3) est réalisé afin de chauffer le milieu d'activation à une température supérieure à 55 et 60 °C, de manière préférée à une température située dans la plage comprise entre environ 130 - 150 °C.

11. Stérilisateur selon l'une quelconque des revendications 7 - 10, **caractérisé en ce que** chaque tête de stérilisateur (3) présente de manière autonome un système de pulvérisation (9) avec une buse de pulvérisation (11) servant à pulvériser du H2O2 dans un flux d'air circulant ensuite à travers le canal de chauffage (7).

12. Stérilisateur selon l'une quelconque des revendications 7 - 11, **caractérisé en ce qu'**un noyau (8) est logé dans le boîtier (6), lequel présente, au niveau de sa périphérie, au moins une rainure formant le canal de chauffage (7).

13. Stérilisateur selon l'une quelconque des revendications 7 - 12, **caractérisé en ce que** le tuyau (26) formant l'ouverture de distribution (27) est fixé, de préférence de manière interchangeable, par une extrémité située de manière éloignée des ouvertures de distribution, au niveau d'une extrémité de la tige (16).

14. Stérilisateur selon l'une quelconque des revendications 7 - 13, **caractérisé par** un système de chauffage (13), par exemple un système de chauffage électrique ou une cartouche de chauffage servant à chauffer le canal de chauffage (7).
